# EUROPEAN PATENT APPLICATION

(11) **EP 2 689 760 A1**
(43) Date of publication of application: **29.01.2014**
(21) Application number: 12177621.5
(22) Date of filing: 24.07.2012
(51) Int. Cl.: A61F 13/49

(54) **Absorbent article**

(71) Applicant: Ontex BVBA, 9240 Zele (BE)
(72) Inventor: Derycke, Tom, 9240 Zele (BE)
(74) Representative: Brants, Johan P.E.

(57) **Abstract**

The present invention provides a disposable absorbent article, suitable to be worn about the lower torso of a wearer, comprising: - a front section, a back section, having oppositely disposed edges and a crotch section, - an absorbent medium disposed on at least said crotch section, and - a pair of laminated stretchable side panels disposed on respective opposite side portions of said back section, each laminated stretchable side panel comprises a layer of elastic material sandwiched between two layers of non-woven material, said layer of elastic material comprises at least two separate elastic zones, a first elastic zone proximal to the absorbent article back section and a second elastic zone distal to the absorbent article back section, said first and second elastic zones extend in the longitudinal direction of the absorbent article.

## Description

### Field of the invention

The present invention relates to absorbent articles, preferably a disposable absorbent article, such as a diaper and more particularly, diapers having stretchable side panels.

### Background

Generally known disposable absorbent articles, such as diapers, training pants, incontinence articles, comprise an absorbent part for covering the wearer's crotch, a pair of side panels extending laterally outwardly from a waist section of the absorbent article, and two fastening means provided on the side edges of the ear parts, respectively. Absorbent articles typically include stretchable materials, such as elastic strands, in the waist region and the cuff regions to provide a snug fit and a good seal of the article. Stretchable materials are also provided in the side panels for easy application and removal of the article and for sustained fit of the article. Stretchable materials have also been used in the side panels for adjustable fit of the absorbent article to the wearer's waist.

Various improvements to the side panels have been proposed to improve the fit of a disposable diaper to the wearer's waist. US 5 685 873 discloses an absorbent article, comprising a front section, a back section, a crotch section and a pair of differentially stretchable ears disposed on respective oppositely disposed side portions of said back section. Each differentially stretchable ear comprises a stretchable inner ear portion and a stretchable outer ear portion having different stretch characteristics either when relaxed or when stretched and fastened. Each stretchable outer ear portion has a lower tension than the stretchable inner ear portions, and each outer portion preferably has an elongation equal to or greater than that of the stretchable inner ear.

WO 2007/069225 discloses a disposable absorbent having a first waist region, a second waist region, and a crotch region interposed between the first waist region and the second waist region. The disposable absorbent article comprises side panels connecting the first waist region to the second waist region. Each side panel comprises three regions including a waist region, a hip region and a leg region wherein each region differs structurally, functionally or visually. Functional differences can include differences in stiffness, bending modulus, elastic modulus, extensibility, force relaxation, set, recovery rate, torsional modulus, compression modulus, compressibility, coefficient of friction, surface energy and combinations thereof. The side panels may be constructed from a number of different materials such as stretch laminates.

WO 2009/082290 discloses an absorbent garment, such as a diaper or an incontinence guard, having a pair of side panels extending laterally outwardly on either side of the garment waist portion. Each side panel has an upper edge and a lower edge and a laterally extending center line there between dividing each side panel or belt member in an upper part and a lower part. At least one of said side panels comprises an elastic member, while the rest of the side panel is of an inelastic material. Said elastic member comprises at least two elastic regions, a first and a second elastic region, each extending over only a part of the length of the respective side panel and over only a part of the distance between the upper and lower edges of the respective side panel. There is a portion of inelastic material between the first elastic region and the lower edge of the side panel. The second elastic region has a main part of its surface area on the lower part of the respective side panel and there is a portion of inelastic material between the second elastic region and the upper edge of the side panel or belt member. The elastic and inelastic regions of the side panels are disposed a way similar to two rows of a chess board. The design and the positioning of the elastic regions within the side panels, in two rows of a chess board, is complex. The manufacture of such side panels is also complex. Moreover, the disposition of the inelastic regions will hamper the elongation of the elastic regions. Hence, longer side panels with more elastic regions are required in order to fit such absorbent articles to a person with a large waist.

In use, the elastic side panels of the absorbent articles described above are bonded to the chassis or to the waist region of the absorbent article. Said side panels are usually provided with releasable fastening means, such as fasteners of the hook and loop type, or adhesive tape fasteners. When placing and fixing the absorbent article around a child waist for instance, the user, such as a mother, is not provided with an indication as to the optimal fitting of the absorbent article neither to the size-fitting of the absorbent article to the waist of the child. These indications are crucial to ensure avoiding leakages and to ensure the comfort of the wearer.

The aim of the present invention is to provide a solution to overcome at least part of the mentioned disadvantages. The invention thereto aims to provide an improved absorbent article with improved side panels properties.

### SUMMARY

The present invention provides a disposable absorbent article, suitable to be worn about the lower torso of a wearer, comprising:
- a front section, a back section, having oppositely disposed edges and a crotch section,
- an absorbent medium disposed on at least said crotch section, and
- a pair of laminated stretchable side panels disposed on respective opposite side portions of said back section,
each laminated stretchable side panel comprises a layer of elastic material sandwiched between two layers of non-woven material, said layer of elastic material comprises at least two separate elastic zones, a first elastic zone proximal to the absorbent article back section and a second elastic zone distal to the absorbent article back section, said first and second elastic zones extend in the longitudinal direction of the absorbent article.

In a preferred embodiment, the first elastic zone (6) has an elastic resistance different than the elastic resistance of the second elastic zone (8).

In a preferred embodiment of the absorbent article, the first elastic zone of the side panels has a higher elastic resistance than the second elastic zone. This is advantageous as it provides the side panels with a strength which prevents tearing of said side panels. In addition, the chances of loosening or removal of the absorbent article after a certain time from wearing it is reduced thanks to the high elastic resistance of the first elastic zone.

In a preferred embodiment of the absorbent article, the first and the second elastic zones of the side panels are separated by a non-elastic zone. This configuration is advantageous as less elastic material is used compared to a side panel wherein only one elastic zone is provided. This is achieved while maintaining a sufficient elongation level of the side panel that ensures a good fit of the absorbent article to the wearer.

In a preferred embodiment of the absorbent article, the first and the second elastic zones represent at least 25% of the surface of each side panel.

In a preferred embodiment of the absorbent article, the first and the second elastic zones extend over the full length (L) of the side panels.

In a preferred embodiment of the absorbent article, the first and/or the second elastic zone of each side panel is provided with a distinction mark. The distinction mark can be visual; can be a sound the user can hear; can be sensorial such as a change in the texture of the side panel which is recognizable by the user's touch or a smell or any combination thereof. The visual distinction mark is selected from the list comprising: colors, textures, patterns, opacity, reflectivity, sheen, presence of drawings or any combination thereof.

In a preferred embodiment, the distinction mark allows to recognize that a threshold of elongation is reached in said first and/or second elastic zone. This is advantageous as it provides the user with a signal indicating that an elongation limit of the first and/or the second elastic zone is reached which avoids tearing of the side panels.

In a preferred embodiment of the absorbent article, a non-elastic zone separates the first elastic zones from the back section. This is advantageous as the elongation of the first elastic zone will be local and will not create a pressure or a strain on the back section of the absorbent article. In the absorbent articles of the prior art, the elastic zone of the side panel is directly bonded to the back sheet. When the side panels are stretched, they will apply a tensile strength on the back section. Said section is in contact with the wearer's waist and will provide him with an uncomfortable feeling along his back and also along his entire waist. This is not the case using the absorbent article of the present invention which provides the wearer with high comfort level as, compared to the absorbent articles of the prior art, a smaller area of his waist is under pressure.

In a preferred embodiment of the absorbent article, the first and the second elastic zones have a shape selected from the list comprising triangular, square and circular shape, preferably said elastic zones have a rectangular shape.

In a preferred embodiment of the absorbent article, the first elastic zone has an elastic resistance from 10 to 80N, preferably from 20 to 70N, more preferably from 30 to 60N or any value comprised in any of the mentioned ranges. In a preferred embodiment of the absorbent article, the second elastic zone has an elastic resistance comprised from 0 to 60N, preferably from 0 to 50N, more preferably from 0 to 40 N or any value comprised in any of the mentioned ranges.

In a preferred embodiment, the side panels are each provided with at least one fastening means.

In a preferred embodiment, the front section of the absorbent article is provided with a marked landing zone suitable for indicating the optimal positioning of the fastening means.

The absorbent article of the present invention provides the user with crucial indications to ensure an optimal fitting and thereby avoid leakages and ensure the comfort of the wearer.

The optimal fitting of an absorbent article is a consequence of the optimal and the symmetrical attachment of the fastening means to the front section of the article. To guarantee an optimal attachment of the fastening means to the front section, equal amounts of strain (also called stress) should be applied on each side panel when fitting the diaper. For an optimal fit and performance of the diaper it is preferred to apply and fit/wear the diaper with lateral symmetry. When different stretching strains are applied on the side panels, the fastening means of each panel will be positioned and attached to the front zone at a random landing location. Moreover and as known, the user is not able to pull simultaneously both side panels and simultaneously attach them to the front section of the absorbent article as the latter has to be maintained in a fixed position during the fitting. The landing locations will be asymmetric and the absorbent article will move to reach an "isoforce" situation wherein a similar stress is applied to each side panel. This creates an asymmetry in the leakage protection features of the absorbent article. The landing zone and the distinction mark of the absorbent article according to the invention ensure an optimal fitting of the article to the wearer. The distinction mark gives an indication as to the stress applied to the side panel. The user is instructed to attach the fastening means when both side panels have identical distinction marks. The landing zone directs the user as to the positioning of the fastening means. The combination of the distinction mark and the landing zone thereby allows an optimal and symmetric attachment of the fastening means to the front section of the article.

The optimal fitting of the absorbent article is also a consequence of the optimal size-fitting of the absorbent article to the waist of the wearer. It is common that the choice of the absorbent article size depends on the weight of the wearer (the baby). The wearer's weight alone cannot provide an optimal size-fitting of the absorbent article. Indeed, the waist circumference is different from a wearer to the other depending on the combination of length, the weight and the slimness or the thickness of the wearer. The absorbent article is big for the wearer if the fastening means of are attached to the front section without applying a stress on each side panel. This leads to a high leakage risk, movement of the absorbent article and even to its complete removal. The absorbent article is small for the wearer if the fastening means of are attached to the front section when applying a high stress on each side panel. This is uncomfortable for the wearer as the elastic side panels will apply a high restoring force (to go back to the unstressed state) on the waist. Using the absorbent article of the prior art, the user will have no indication as to the optimal size-fitting. The distinction marks of the side panels of the absorbent article according to the present invention provide said indication and allow the user to easily and with certainty decide as to changing the size of the used or of the next to be used absorbent article. Further, the distinction marks warns the user when a certain elongation threshold is reached in one or both side panels. In this case, the chances of damages of one or both side panels are lowered which also lowers the leakage and/or to the complete damage of the absorbent article and its replacement by another non-used absorbent article.

### DESCRIPTION OF THE FIGURES

The invention will be further described in detail with reference to the exemplary embodiments represented by the accompanying figures, in which
**FIG. 1** shows a plan view of an embodiment of an absorbent article according to the present invention.
**FIG. 2** shows a plan view of another embodiment of an absorbent article according to the present invention.
**FIG. 3** shows is a perspective view of the absorbent article shown in **Fig.2****.**
**FIG. 4** **a** shows an embodiment of a side panel of an absorbent article according to the present invention wherein the elastic zones are separated by a non-elastic zone **b** shows another embodiment of a side panel of an absorbent article wherein the elastic zones are not separated by a non-elastic zone **c** shows an embodiment of a side panel of an absorbent article of the present invention wherein the elastic zones are not separated by a non-elastic zone **d** shows another embodiment of a side panel having three elastic zones of different elastic resistance.
**FIG. 5** shows a cross section view of a side panel according to line I-I of **Fig. 1**
**FIG. 6** shows a cross section view of the a first embodiment of the absorbent article according to line II-II of **Fig. 1**
**FIG. 7** shows a cross section view of a second embodiment of the absorbent article according to line II-II of **Fig. 1**
**FIG. 8** shows a cross section view of a third embodiment of the absorbent article according to line II-II of **Fig. 1**
**FIG. 9** shows a cross section view of the a fourth embodiment of the absorbent article according to line II-II of **Fig. 1**
**FIG. 10** is a front view of a preferred embodiment of the present invention being initially fitted onto the wearer such as the fastening means are not yet attached to the absorbent article.
**FIG. 11** is a front view of a preferred embodiment of the present invention being completely fitted onto the wearer such as the fastening means are attached to the absorbent article.
**FIG. 12** shows the evolution, according to the applied stress, of a side panel elongation. The side panel comprises two different elastic zones.

### DETAILED DESCRIPTION OF THE INVENTION

The present invention relates to an absorbent article having at least two elastic side panels with improved properties. The absorbent article of the present invention can be a diaper used for babies, children or any adult person who is in need of such article, for instance a sick person or an old person.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "% by weight" (weight percent), here and throughout the description unless otherwise defined, refers to the relative weight of the respective component based on the overall weight of the formulation.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

As used herein, the term "diaper" refers to an absorbent article generally worn by infants and incontinent persons about the lower torso.

By "stretch", it is meant that the material has the ability to extend beyond its original size in at least one dimension when subjected to a tensile force (i. e., tension) applied in the direction of that dimension. "Stretch" may be unidirectional, bi-directional, or multi- directional. The specific "stretch" properties of a material may vary along any of the stretch vectors. As used herein, stretch includes both plastic and elastic deformation. The term "elastic" or "elastomeric" as used herein refers to any material that upon application of a biasing force, can stretch to an elongated size of at least about 125 percent of its relaxed, original size, without rupture or breakage, and upon release of the applied force, recovers at least about 40% of its elongation, preferably recovers at least 60% of its original size, most preferably recovers about 80% of its original size.

The term "non-elastic" or "inelastic" are used herein as synonyms and they refer to any material that does not fall within the definition of "elastic" above.

As used herein, the term "elastic resistance" describes an elastic force that tends to resist an applied tensile force causing a material provided therewith to tend to contract to an untensioned configuration in response to a stretching force.

"Longitudinal" is a direction running parallel to the maximum linear dimension of the article and includes directions within t45 of the longitudinal direction. The "lateral" or "transverse" direction is orthogonal to the longitudinal direction.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

The term "nonwoven" as used herein refers to a manufactured sheet, web, or batt directionally or randomly oriented fibres bonded by friction and/or cohesion and/or adhesion, excluding paper and products that are woven, knitted, tufted stitchbonded incorporating binding yarns or filaments, or felted by wet-milling, whether or not additionally needled. The fibres may be of natural or man-made origin. They may also be staple or continuous filaments or be formed in situ.

The absorbent article of the present invention comprises a liquid permeable top sheet, a liquid impermeable back sheet, and an absorbent medium disposed between the top sheet and the back sheet. The article may also include one or more features such as, but not limited to, ears or side panels, leg cuffs, fastener components and/or a belt. The top sheet, back sheet and the absorbent medium could be made from any suitable material known to the person skilled in the art.

The term "top sheet" refers to a liquid permeable material sheet forming the inner cover of the absorbent article and which in use is placed in direct contact with the skin of the wearer. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The top sheet material may further be composed of two fibres, which may be bonded to each other in a bonding pattern, as e.g. disclosed in EP-A- 1 035 818. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g. urine or menstrual fluid. The inner coversheet may further be different in different parts of the absorbent article.

The "back sheet" refers to a material forming the outer cover of the absorbent article. The back sheet may be the same or different in different parts of the absorbent article. At least in the area of the absorbent medium the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials.

The "absorbent medium" is the absorbent structure disposed between the top sheet and the back sheet of the absorbent article in at least the crotch region thereof. The absorbent material can be of any conventional kind. Examples of commonly occurring absorbent materials are cellulosic fluff pulp, tissue layers, highly absorbent polymers (so called superabsorbents), absorbent foam materials, absorbent nonwoven materials or the like. It is common to combine cellulosic fluff pulp with superabsorbent polymers in an absorbent material. Superabsorbent polymers are water-swellable, water-insoluble organic or inorganic materials capable of absorbing at least about 20 times their own weight of an aqueous solution containing 0.9 weight percent of sodium chloride. Organic materials suitable for use as superabsorbent materials can include natural materials such as polysaccharides, polypeptides and the like, as well as synthetic materials such as synthetic hydrogel polymers. Such hydrogel polymers include, for example, alkali metal salts of polyacrylic acids, polyacrylamides, polyvinyl alcohol, polyacrylates, polyvinyl pyridines, and the like. Other suitable polymers include hydrolyzed acrylonitrile grafted starch, acrylic acid grafted starch, and isobutylene maleic anhydride copolymers and mixtures thereof. The hydrogel polymers are preferably lightly cross-linked to render the material substantially water insoluble. Preferred superabsorbent materials are further surface cross-linked so that the outer surface or shell of the superabsorbent particle, fibre, flake, sphere, etc. possesses a higher crosslink density than the inner portion of the superabsorbent. The superabsorbent materials may be in any form which is suitable for use in absorbent composites including particles, fibres, flakes, spheres, and the like.

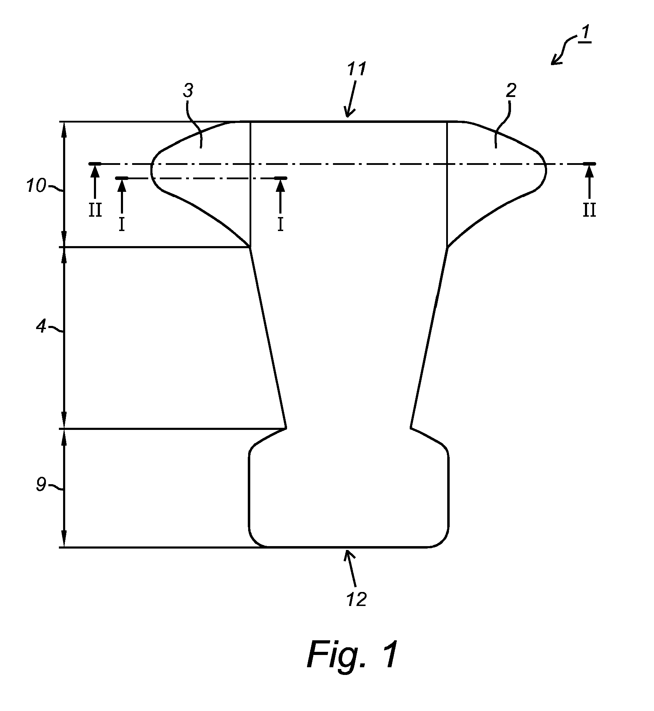

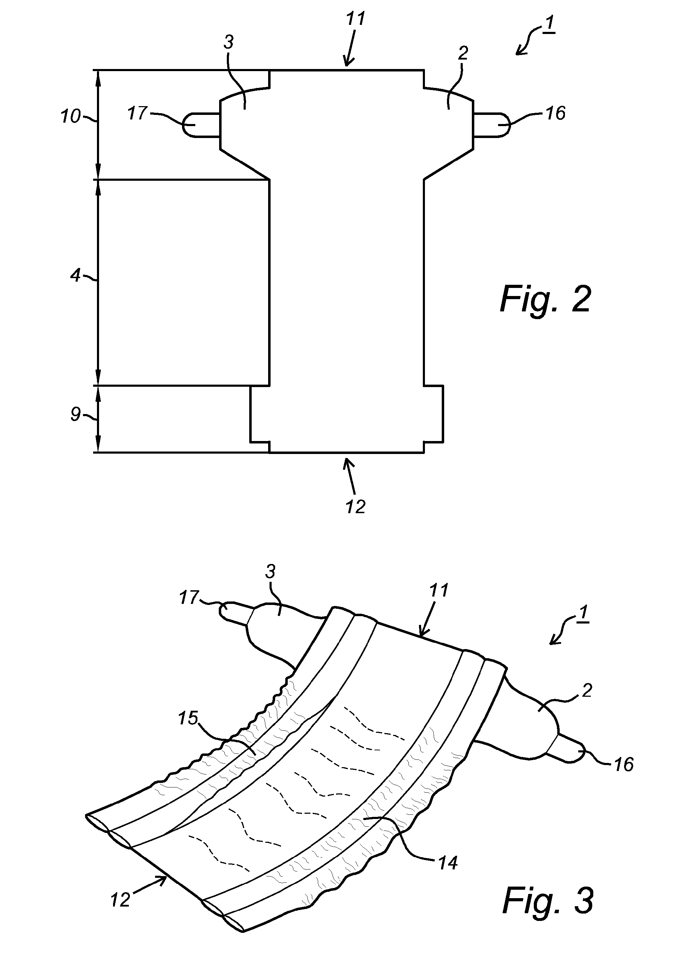

**Fig. 1** and **2** show two embodiments of an absorbent article according to the present invention. The absorbent article of **Fig. 1** is devoid of leg cuffs while the absorbent article of **Fig. 2** is provided with leg cuffs **14, 15.** Said leg cuffs **14, 15** are also visible in **Fig. 3** wherein a detailed perspective view of the absorbent article of **Fig. 2** is shown. The side panels of **Fig. 2** and **Fig. 3** are also provided with fastening means **16, 17** used to fasten and fit the absorbent article around a wearer's waist.

In a first aspect, the present invention provides a disposable absorbent article, suitable to be worn about the lower torso of a wearer. The absorbent article comprises:
- a front section **9,** a back section **10,** having oppositely disposed edges **11, 12** and a crotch section **4 (****Fig. 1** and **Fig. 2****).** The back section **10** might be provided with an elastic material for a better fit of the absorbent article around the wearer's waist.
- an absorbent medium disposed on at least said crotch section **4,** and,
- a pair of laminated stretchable side panels **2, 3** disposed on respective opposite side portions of said back section **4 (****Fig. 1** and **2****).**

In a preferred embodiment, the side panels **2, 3** of the absorbent article of the invention are each provided with at least one releasable fastening means **16, 17 (****Fig. 2** and **Fig 3****).** Said releasable fastening means are preferably in the form of mechanical fasteners of the hook and loop type, or adhesive tape fasteners. In a preferred embodiment, a single loop panel or a pair of loop panels is joined to the impermeable back sheet of the front section while a hook material is placed on each side panel. Said hook material is placed on the side panel surface which is in the same plane as the top sheet when the absorbent article is placed on a flat surface as shown in **Fig. 1** and **2****.** The loop and the hook may be of any material provided they are compatible with each other such as to allow fixation of the absorbent article around a wearer's waist.

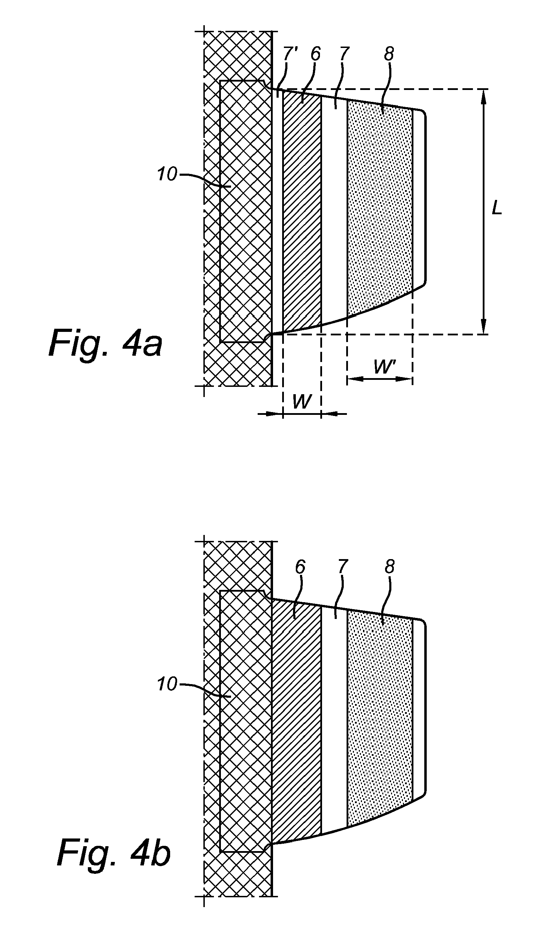

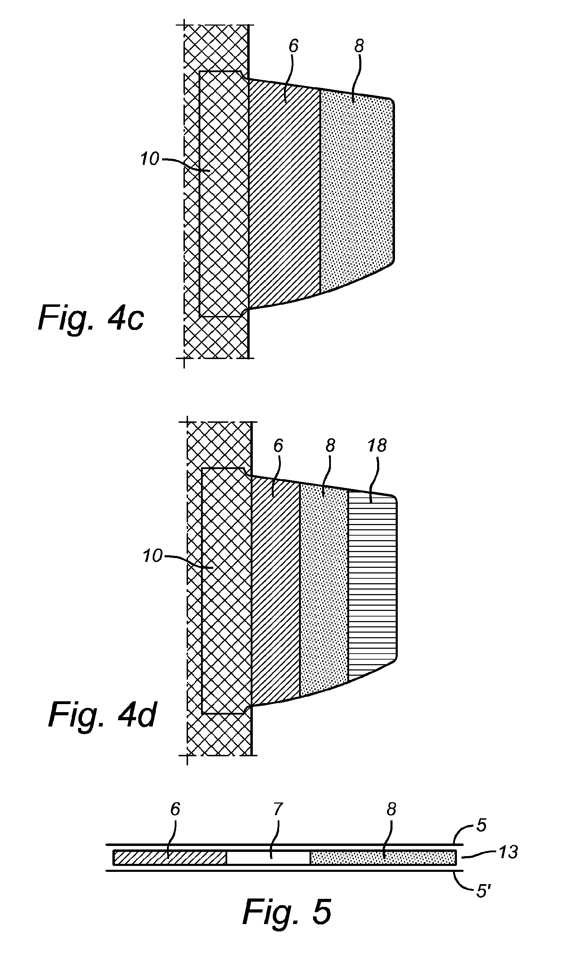

**Fig. 5** shows a cross section view of an embodiment of side panel according to the present invention according to line I-I shown in **Fig. 1****.** Each laminated stretchable side panel **2, 3** comprises a layer of elastic material **13** sandwiched between two layers of non-woven material **5, 5'.** Said layer of elastic material **13** comprises at least two separate elastic zones, a first elastic zone **6** (hatched area in **Fig. 4a****)** proximal to the absorbent article **1** back section **10** (cross hatched area in **Fig. 4a****)** and a second elastic zone **8** (area filled with dots in **Fig. 4a****)** distal to the absorbent article **1** back section **10.** Said first and second elastic zones extend in the longitudinal direction of the absorbent article **1 (****Fig. 4a****).**

In a preferred embodiment, each section of the absorbent article might comprise a liquid permeable top sheet and/or a liquid impermeable back sheet and/or an absorbent medium disposed between the top sheet and the back sheet.

A "hook-and-loop fastener" refers to complementary fastening means having a "hook" portion and a "loop" portion and which are refastenable. The term "hook" as used herein refers to any element capable of engaging another element, the so called "loop" portion. The term "hook" is not limited to only "hooks" in its normal sense, but rather encompasses any form of engaging elements, whether unidirectional or bi-directional. The term "loop" is likewise not limited to "loops" in its normal sense, but also encompasses any structure capable of engaging with a "hook" fastener. Examples of "loop" materials are fibrous structures, like nonwoven materials.

The side panels **2, 3** can have any shape such as but not limited to square, rectangular, triangular, circular and trapezoidal shape. The side panels **2, 3** can be joined to the respective opposite side portions of the back section **4,** by a known method, such as heat-sealing or adhesive bonding.
The side panels **2, 3** may also be formed integrally with the back section **4** by projecting and joining together the respective top sheet and/or back sheet and/or absorbent medium outward in lugs having the shape of the side panels **2, 3.**

Preferably, the side panels **2, 3** are formed by laminating a layer of nonwoven fabric **5,** a layer of thermoplastic film **5'** and a layer of elastic material **13.** The layer of elastic material might be sandwiched between the nonwoven fabric layer **5** and the thermoplastic film **5'** by adhesive layers. The layer of nonwoven fabric might be made of natural fibers, synthetic fibers or a blend of natural fibers and synthetic fibers. The layer of thermoplastic film might be made of polyethylene or polypropylene.

The thickness of each non-woven material **5, 5'** layer is comprised between 0.05 and 4 mm, preferably between 0.1 and 3 mm, more preferably between 0.2 and 2 mm. The thickness of the layer of elastic material is comprised between 0.1 and 5 mm, preferably between 0.2 and 4 mm, more preferably between 0.3 and 3 mm. The total thickness of the each side panel is comprised between 0.2 and 7 mm, preferably between 0.25 and 6 mm, more preferably between 0.3 and 5 mm.

In a preferred embodiment, the elastic zones of each side panel have different elastic properties. In a preferred embodiment, the first elastic zone **6** has a higher elastic resistance than the second elastic zone **8.** In a preferred embodiment, the ratio: elastic resistance of the first elastic zone **6** to the elastic resistance of the second elastic zone **8** is from 0.1 to 0.9, preferably from 0.2 to 0.8, more preferably from 0.3 to 0.7, even more preferably from 0.4 to 0.6 or any value comprised within the mentioned ranges. In a further preferred embodiment, the first elastic zone **6** has an elastic resistance from 10 to 80N, preferably from 20 to 70N, more preferably from 30 to 60N or any value comprised in any of the mentioned ranges. The second elastic zone **8** has an elastic resistance comprised from 0 to 60N, preferably from 0 to 50N, more preferably from 0 to 40 N or any value comprised in any of the mentioned ranges.

In a preferred embodiment, the ratio between the elastic resistance of the elastic zone which is proximal to the absorbent article back section (herein called the first elastic zone) and the elastic resistance of the elastic zone which is distal to the to the absorbent article back section (herein called the second elastic zone) is from 1.1 to 2, preferably from 1.2 to 1.9, more preferably from 1.3 to 1.8, even more preferably from 1.4 to 1.7, most preferably from 1.5 to 1.6 or any value comprised in between the mentioned ranges.

The elastic resistance is measured on cut out sections from each elastic zone of the side panels. The measurements are performed using a test rig that comprises a vertical rectangular base plate provided with several spaced clamps. Said cut out sections have an equal surface of 5 cm². Each cut out section is clamped to the test rig at one end and a weight is clamped to the opposed free end. Each cut out section is clamped such as the weight applies a stress that elongates the cut out sections in the same direction as the elongation direction of the side panels of the absorbent article. The weight is slowly released until it applies a tension to the cut out section. The weight is then allowed to hang freely. The weight and clamp together apply a force of 35 N to the cut out sections. As soon as the weight is allowed to hang freely, a timer is started. After a certain elapsed time, the cut out sections are released from the clamps and their length is measured. Said elapsed time is at least 60 min. The elastic resistance is measured for at least three cut out sections from each elastic zone. In order to avoid the influence of aging of the articles on the test results, the articles should be no more than 6 months old, i. e. the test is to be performed on articles which have been manufactured during the past six months.

In a preferred embodiment, the first and the second elastic zones **6, 8** are separated by a non-elastic zone **7 (****Fig. 5****).** In a preferred embodiment, said non-elastic zone is made of a material that does not stretch upon application of a biasing force. The non-elastic zone is made of a material that stretches to an elongated size of maximum 125%, preferably 115%, more preferably 105% of its relaxed, original size.

In a preferred embodiment, the surface of both the first and the second elastic zones **6, 8** represents at least 40%, preferably at least 50%, more preferably at least 60%, even more preferably at least 70%, most preferably at least 80%, even most preferably at least 90%, or any value in between, of the surface of each side panel **2, 3.**

In a preferred embodiment, the first and the second elastic zones **6, 8** extend over the full length **(L,** **Fig. 4a****)** of the side panels **2, 3.** The full length of a side panel is the distance between two opposite edges of the side panel measured in a direction which is parallel to the longitudinal direction of the absorbent article. It is to be understood that the length of the side panel can vary depending on the side panel shape. For instance said length will be constant if the side panel have a rectangle or a square shape while it will be non-constant if the side panel has a trapezoidal shape.

In a preferred embodiment, the length **L (****Fig. 4a****)** of the first and the second elastic zones **6, 8** is comprised between 0.2 and 8 cm, preferably between 0.3 and 7 cm, more preferably between 0.4 and 6 cm, most preferably between 0.5 and 5 cm or any value comprised in between these ranges. It is to be understood that **L** can vary within the same side panel if the edges of said side panel are not parallel to each other **(****Fig. 4a, b****,** **c and d****).** Here, the edges of the side panel are those which are not extending in the same longitudinal direction of the absorbent article. In a further preferred embodiment, the second elastic zone has low elastic resistance compared to the first elastic zone as its surface is smaher than the surface of the first elastic zone.

In a preferred embodiment, the width **W** of the first elastic zone **6** is comprised between 0.5 and 5 cm, preferably between 1 and 4 cm, more preferably between 1.5 and 3 cm.

In a preferred embodiment, the width **W (****Fig. 4a****)** of the first elastic zone **6** is equal to the width **W' (****Fig. 4a****)** of the second elastic zone **8.**

In a preferred embodiment, the width **W (****Fig. 4a****)** of the first elastic zone **6** is smaller than the width **W' (****Fig. 4a****)** of the second elastic zone **8.** In a further preferred embodiment, the width **W (****Fig. 4a****)** of the first elastic zone **6** is at least 5%, preferably 10%, more preferably 15% smaller than the width **W' (****Fig. 4a****)** of the second elastic zone **8.** In a further preferred embodiment, the ratio **W/W'** of the width of the first elastic zone **6** to the width of the second elastic zone **8** is at least 0.01, 0.1, 0.2, 0.3, 0.4, 0.5, 0.6, 0.7, 0.8, 0.9, 0.95, 0.96, 0.97, 0.98, 0.99 or any value comprised within the mentioned values.

In another preferred embodiment, the width **W (****Fig. 4a****)** of the first elastic zone **6** is higher than the width **W' (****Fig. 4a****)** of the second elastic zone **8.** In a further preferred embodiment the width **W (****Fig. 4a****)** of the first elastic zone **6** is at least 5%, preferably 10%, more preferably 15% higher than the width **W' (****Fig. 4a****)** of the second elastic zone **8.** In a further preferred embodiment, the ratio **W/W'** of the width of the first elastic zone **6** to the width of the second elastic zone **8** is at least 1.01, 1.1, 1.2, 1.3, 1.4, 1.5, 1.6, 1.7, 1.8, 1.9, 1.95, 1.96, 1.97, 1.98, 1.99 or any value comprised within the mentioned values.

In a preferred embodiment, the first and/or the second elastic zone **6, 8** is provided with a distinction mark. In a further preferred embodiment, the distinction mark allows the user to recognise when a threshold of elongation is reached in said first and/or second elastic zone **6, 8.** In a preferred embodiment, the distinction mark is noticeable by the user when the first elastic zone reaches at least 140%, preferably at least 150%, more preferably at least 160% of its relaxed, original size. Here, the size of the elastic zone is measured in the same direction as the direction of the applied force or strain.

In a preferred embodiment, each side panel is suitable to be elongated to a size of at least 110%, preferably at least 120%, more preferably at least 130% of its relaxed, original size. Also here, the size of the elastic zone is measured in the same direction as the direction of the applied force or strain.

In a preferred embodiment, within the same side panel, the first and the second elastic zone **6, 8** might be provided with similar distinction marks.

In another preferred embodiment, within the same side panel, the first and the second elastic zone **6, 8** might be provided with different distinction marks. In a further preferred embodiment, the distinction mark can be visual; can be a sound the user can hear; can be sensorial such as a change in the texture of the side panel which is recognizable by the user's touch or a smell or any combination thereof. The visual distinction mark is selected from the list comprising: colors, textures, patterns, opacity, reflectivity, sheen, presence of drawings or any combination thereof.

In one embodiment, elastic materials can be prestrained and attached to the first and to the second elastic zones at different levels of strain producing different visible gathers or rugosities to further distinguish the appearance of each zone while at the same time provide elastic properties desirable for each of the zone.

The distinction mark can be combined with the different elastic zones of the side panel in order to a) provide an indication of the stretchability and the elongation level reached at a certain time and under a certain applied strain b) of where to grasp the side panel in order to stretch it during application, c) provide a visually appealing pattern (e.g., racing stripes, etc.) to a child wearer, d) provide an indication of the front vs. the back of the article, e) provide intuitive instructions to the caregiver to facilitate application or removal of the article, and/or f) coordinate with other graphics on the front section of the absorbent article.

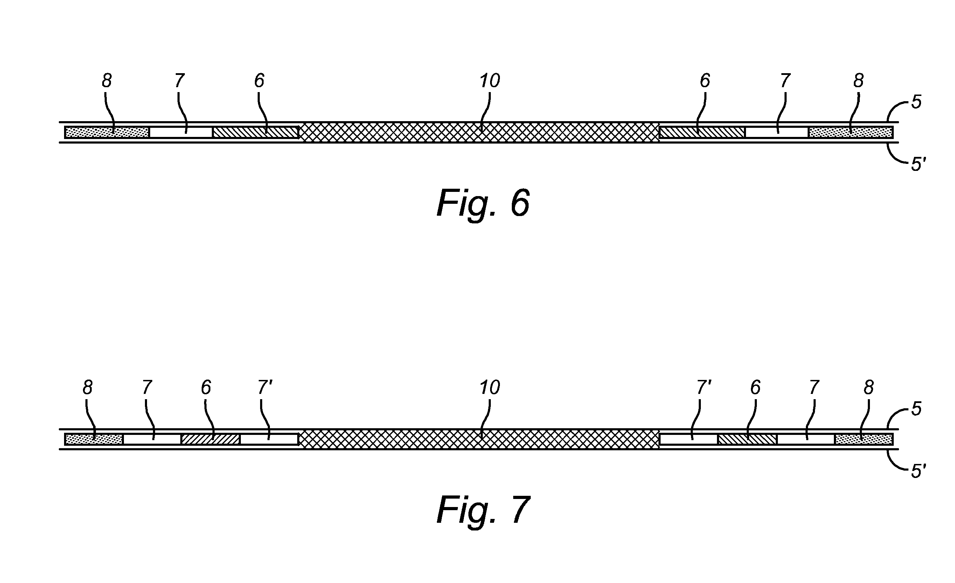

In a preferred embodiment of the absorbent article of the present invention, each side panel further comprises an extra non-elastic zone **7'** which separates the first elastic zones **6** from the back section **10.** The extra non-elastic material **7'** can be made of the same material or from a different material as the non-elastic zone **7** which separates the first elastic zone **6** and the second elastic zone **8 (****Fig. 4a****).** **Fig. 7** shows a cross sectional view of the absorbent article **1** according to line II-II shown in **Fig. 1****.** The back section **10** of the diaper is illustrated by the cross hatched area which is followed by the extra non-elastic zone **7'** (blank area) which is subsequently followed by the first elastic zone **6** (hatched area), the non-elastic zone **7** and the second elastic zone **8.** The elastic resistance of the extra non-elastic region **7'** is at least 25N.

In another preferred embodiment of the absorbent article, the first elastic zone **6** is directly attached to the back section **10** as shown in **Fig. 4b** and in **Fig. 6****.** In this embodiment, the back section **10** of the diaper is illustrated by the cross hatched area which is followed by the first elastic zone **6** (hatched area), the non-elastic zone **7** and the second elastic zone **8.**

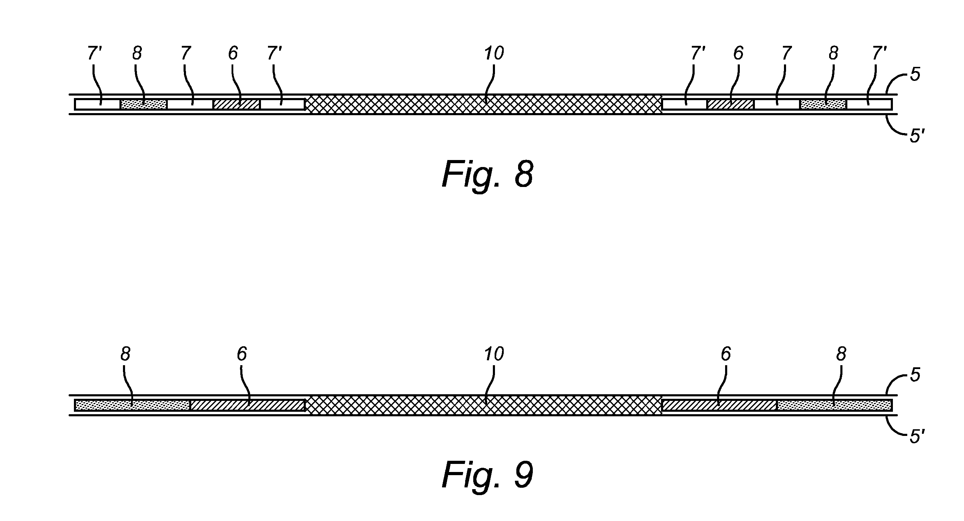

In another preferred embodiment of the absorbent article, shown in **Fig. 8****,** the back section **10** of the absorbent article (cross hatched area) is followed by the extra non-elastic zone **7'** (blank area) which is subsequently followed by the first elastic zone **6** (hatched area), the non-elastic zone **7,** the second elastic zone **8** and another non elastic zone **7".**

**Fig. 4c** shows another preferred embodiment of the absorbent article. In this embodiment, the back section **10** of the absorbent article (cross hatched area) is adjacent to the first elastic zone **6** (hatched area) which is directly followed by the second elastic zone **8.** **Fig. 9** shows a cross sectional view of the absorbent article **1** according to line II-II shown in **Fig. 1****.** The back section **10** of the diaper is followed by the first elastic zone **6** (hatched area) which is followed adjacent to the second elastic zone **8.**

**Fig. 4d** shows another preferred embodiment of the absorbent article. In this embodiment, the side panel is provided with three zones **6, 8, 18** having different elastic resistance. The three elastic zones can be directly jointed to each other as presented in **Fig. 4d****.** The three elastic zones can also be separated by one or more non-elastic zones (not shown). The back section **10** of the absorbent article is adjacent to the first elastic zone **6** which is directly followed respectively by the second elastic zone **8** and the third elastic zone **18.** The elastic resistance of the first elastic zone **6** is higher than the elastic resistance of the second elastic zone **8** which is higher than the elastic resistance of the third elastic zone **18.**

In a preferred embodiment, the first and the second elastic zones and/or the non-elastic zones have a shape selected from the list comprising triangular, square and circular shape, preferably said elastic and/or non-elastic zones have a rectangular shape.

In a preferred embodiment, the different zones of the side panels of the absorbent article according to the present invention are obtained by co-extrusion of different films. Said films are glued to each other. The gluing pattern might influences the elasticity of the different elastic zones such as the more glue used for the production of the elastic zone, the more elastic resistant said zone will be.

The absorbent article of the present invention is provided with at least one landing zone which is provided in the front section. Said landing zone indicates the position in which each fastening means should be attached to the front section of the absorbent article. This ensures an optimal and symmetric attachment of the fastening means to the front section of the article. The front section of the absorbent article can be provided with a single one landing zone for both fastening means. The front section of the absorbent article can also be provided with at least two landing zones; one landing zone for each fastening means.

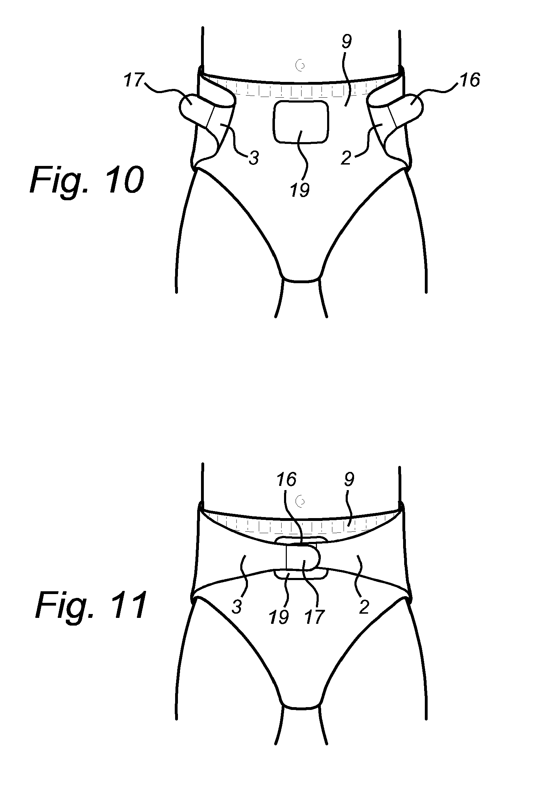

**Fig. 10** shows an embodiment in which an absorbent article of the present invention is initially fitted onto the wearer such as the fastening means **16, 17** of the side panels **2, 3** are not yet attached to the absorbent article. The front section **9** of the absorbent article is provided with a landing zone **19** which indicates the optimal attachment location of the fastening means to the front section **9.** **FIG. 11** shows the absorbent article being completely fitted onto the wearer's waist. The fastening means **16, 17** are attached to the landing zone **19.** It is to be understood that depending on how large is the wearer's waist, the fastening means will be either in contact with each other as shown in **Fig. 11** or not in contact with each other (not shown).

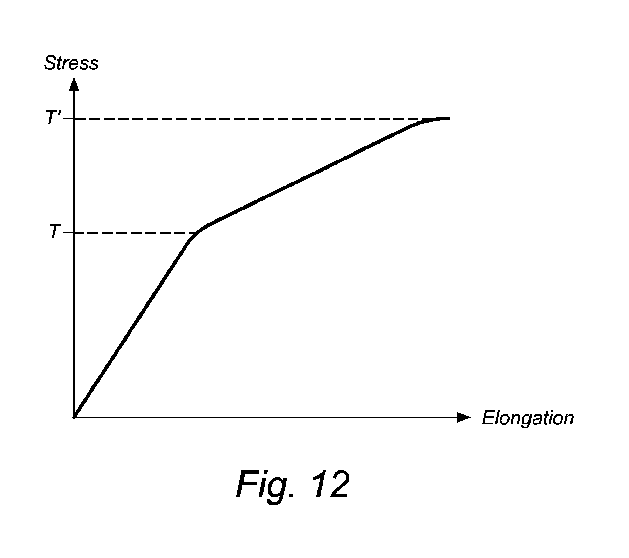

In use and after being placed around a wearer's waist as known to a person skilled in the art, the user pulls a side panel and attaches it, using its fastening means, to the frontal section of the absorbent article than pulls and attaches the other side panel to said frontal section. Using the absorbent article of the present invention, when the user pulls one of the side panels, the second elastic zone **8** is the first zone to elongate until reaching an elongation threshold **T (****Fig. 12****).** The correlation between the applied stress and the elongation of the second elastic zone is linear or substantially linear. At least one distinctive mark will be visible to the user when **T** is reached. Said distinctive mark can also progressively become visible to the user. For instance, the higher is the stress applied by the user on the side panel; a certain colour or a certain image of the second zone **8** will be more and more apparent. Also when **T** is reached, the elongation of the first elastic zone **6** starts as shown in **Fig. 12****.** Similarly, at least one distinctive mark will be visible to the user when the maximum elongation **T'** of the first elastic zone is reached or almost reached. Said distinctive mark can progressively become visible to the user. For instance, the higher is the stress applied by the user on the side panel; a certain colour and/or a certain image of the second zone **8** will be more and more apparent. The distinctive mark can be any of those listed above or any combination thereof.

In another embodiment, when the user pulls one of the side panels, the second elastic zone **8** is the first zone to elongate until reaching a certain elongation length **EL.** The correlation between the applied stress and the elongation of the second elastic zone is linear or substantially linear. At the same stretch force applied to reach **EL,** the elongation of the side panel continues until reaching an elongation length **EL1.** At least one distinctive mark will be visible to the user when **EL** and/or **EL1** is reached. Said distinctive mark can also progressively become visible to the user. For instance, the higher is the stress applied by the user on the side panel; a certain colour or a certain image of the second zone **8** will be more and more apparent. When **EL1** is reached, the elongation of the first elastic zone **6** starts until reaching an elongation length **EL2.** The correlation between the applied stress and the elongation of the second elastic zone is linear or substantially linear. At the same stretch force applied to reach **EL2,** the elongation of the side panel continues until reaching an elongation length **EL3** which is the maximum elongation length of the first elastic zone and hence of the side panel. Similarly, at least one distinctive mark will be visible to the user when **EL2** and/or **EL3** is reached or almost reached. Said distinctive mark can progressively become visible to the user. For instance, the higher is the stress applied by the user on the side panel; a certain colour and/or a certain image of the second zone **8** will be more and more apparent. The distinctive mark can be any of those listed above or any combination thereof.

In another embodiment, one or more ink-jet printed markers are provided on the elastic material and/or the non-woven material of each side panel. Said markers have preferably a line shape and are provided such as to delimit, in the longitudinal direction, each elastic zone. In use and after being placed around a wearer's waist, the user pulls each side panel and attaches it, using its fastening means, to the frontal section of the absorbent article. The distance between two successive ink-jet printed markers provides an indication to the user as to the elongation of each side panel. The user will be instructed to attach the absorbent article to the wearer's waits such as to have, within each side panel, similar distances separating the ink-jet printed markers.

In another embodiment, several stripes are provided on the elastic material and/or the non-woven material of each side panel. Said stripes are positioned in the longitudinal direction of the side panel. In use and after being placed around a wearer's waist, the user pulls each side panel and attaches it, using its fastening means, to the frontal section of the absorbent article. The distance between the stripes of each side panel provides an indication to the user as to the elongation of each side panel. The user will be instructed to attach the absorbent article to the wearer's waits such as to have, within each side panel, similar distances between said stripes.

In another embodiment, the elastic material and/or the non-woven material of one or both side panels has in cross sectional view, at least one "Z" structure. The cross sectional view is according to a line parallel to lines I-I and II-II. Preferably each elastic zone in each side panel has in cross sectional view, at least one "Z" structure. In said structure, the section connecting the two parallel sections of the "Z" is marked. Said marked section can be a printed line on the elastic film, said line can have a different colour than the elastic film itself. In use and after being placed around a wearer's waist, the user pulls each side panel and attaches it, using its fastening means, to the frontal section of the absorbent article. When the second elastic zone is stretched to the maximum, its "Z" structure becomes flat and the marked section of said "Z" structure becomes visible to the user. If needed, the user will further stretch the side panel and when the first elastic zone is stretched to the maximum, its "Z" structure becomes flat and the marked section becomes visible, thereby providing the user with indications as to the elongation of each elastic zone.

In another embodiment, the elastic material of each side panel is provided with at least one printed line or zone. Said printed line or zone might have a distinguishable colour and is positioned in the first elastic zone. Each side panel further comprises a spunbond cover which extends from the diaper chassis and is positioned such as to cover the printed line. In use and after being placed around a wearer's waist, the user pulls each side panel and attaches it, using its fastening means, to the frontal section of the absorbent article. When the first elastic zone is stretched to the maximum, the printed line is no longer hidden by the spunbond cover and becomes visible to the user. It is to be understood that the spunbond cover and the elastic zones might be sandwiched between two non-woven layers. Another option is that only the elastic zones are sandwiched between two non-woven layers while the spunbond is positioned at the outer surface of the side panel, said surface remains visible even when the diaper is worn by a wearer.

In another embodiment, the elastic material is provided with more than one printed line. Said printed lines are positioned within the second and the first elastic zones and can have similar colours, however it is preferable that they have different colours. Each side panel further comprises a spunbond cover which extends from the diaper chassis and is positioned such as to cover the printed line which is proximal to said diaper chassis. Each printed line becomes visible under a certain applied stress. For a symmetric optimal attachment of the absorbent article to a wearer's waist, the same number of printed lines having the same colours should be visible to the user on each side panel. The printed line covered by the spunbond provides an indication as to the maximal elongation of the side panel elastic zones. For instance, when the printed line covered by the spunbond becomes visible, the user is advised to use a diaper of a bigger size. It is to be understood that the spunbond cover and the elastic zones might be sandwiched between two non-woven layers. Another option is that only the elastic zones are sandwiched between two non-woven layers while the spunbond is positioned at the outer surface of the side panel, said surface remains visible even when the diaper is worn by a wearer.
In another embodiment, a glue pattern is provided on the elastic material of each side panel. Said glue pattern is discontinuous, i.e. having intermittent glue zones and non-glue zones. Said glue pattern is also non-uniform along the elastic material. The glue is positioned at one side, preferably, on both sides of the elastic material. The glue pattern of the elastic material proximal to the diaper chassis shows glue zones that are larger and/or less spaced compared to the glue zones of the glue pattern of the zone distal to the diaper chassis. Thus, the elastic material proximal to the diaper chassis has a lower elasticity compared to the elastic material distal to the diaper chassis.

In another embodiment, the elastic material is provided with at least one marked zone such as a coloured zone. Said marked zone might be provided in one or in each elastic zone of the side panel. Said marked zone is covered by at least one or two opaque zones provided in the non-woven layers sandwiching said elastic material. The opaque zones might have an equivalent or a bigger size compared to the marked zone. In use and after being placed around a wearer's waist, the user pulls each side panel and attaches it, using its fastening means, to the frontal section of the absorbent article. When the second elastic zone is stretched to the maximum, the marked zone is no longer hidden by the opaque zones and becomes visible to the user. If required the user further stretches the side panel and when the first elastic zone is stretched to the maximum, the marked zone is no longer hidden by the opaque zones and becomes visible to the user. In another option, when the second elastic zone is stretched to the maximum, the opaque zones become transparent thereby making the marked zone visible to the user. If required the user further stretches the side panel and when the first elastic zone is stretched to the maximum, the opaque zones become transparent thereby making the marked zone visible to the user.

For absorbent articles of the present invention wherein each side panel comprises three or more elastic zones, it is the most distal zone from the article's chassis that have the least elastic resistance. Said elastic resistance increases in each zone until reaching the elastic zone which is most proximal to the article's chassis or back section. Said most proximal zone to the article's chassis have the highest elastic resistance. The zone having the least elongation resistance is the first zone to elongate until reaching an elongation threshold. Then, the adjacent zone having higher elastic resistance starts to elongate until reaching its elongation threshold. Then, the adjacent zone having even higher elastic resistance starts to elongate until reaching its elongation threshold.
Although the present invention has been described with reference to preferred embodiments thereof, many modifications and alternations may be made by a person having ordinary skill in the art without departing from the scope of this invention which is defined by the appended claims

## Claims

1. A disposable absorbent article (1), suitable to be worn about the lower torso of a wearer, comprising:
- a front section (9), a back section (10), having oppositely disposed edges (11, 12) and a crotch section (4),
- an absorbent medium disposed on at least said crotch section (4), and
- a pair of laminated stretchable side panels (2, 3) disposed on respective opposite side portions of said back section (4),
each laminated stretchable side panel (2, 3) comprises a layer of elastic material (13) sandwiched between two layers of non-woven material (5, 5'), said layer of elastic material (13) comprises at least two separate elastic zones, a first elastic zone (6) proximal to the absorbent article (1) back section (10) and a second elastic zone (8) distal to the absorbent article (1) back section (10), said first and second elastic zones extend in the longitudinal direction of the absorbent article (1).

2. The absorbent article (1) according to claim 1, wherein the first elastic zone (6) has an elastic resistance different than the elastic resistance of the second elastic zone (8).

3. The absorbent article (1) according to any of claims 1 or 2, wherein the ratio between the elastic resistance of the first elastic zone and the elastic resistance second elastic zone is from 1.1 to 2.

4. The absorbent article (1) according to any of claims 1 or 3, wherein the first and the second elastic zones (6, 8) are separated by a non-elastic zone (7).

5. The absorbent article (1) according to any of claims 1-4, wherein the first and the second elastic zones (6, 8) represent at least 25% of the surface of each side panel (2, 3).

6. The absorbent article (1) according to any of claims 1-5, wherein the first and the second elastic zones (6, 8) extend over the full length (L) of the side panels (2, 3).

7. The absorbent article (1) according to any of claims 1-6, wherein the first and/or the second elastic zone (6, 8) is provided with a distinction mark.

8. The absorbent article (1) according to claim 7, wherein said distinction mark is selected from the list comprising: colors, textures, patterns, opacity, reflectivity, sheen, presence of drawings or any combination thereof.

9. The absorbent article (1) according to any of claims 1-8, wherein each side panel is provided with at least one fastening means.

10. The absorbent article (1) according to any of claims 1-9, wherein the front section (9) is provided with a landing zone (19) for attaching the fastening means (16, 17).

11. The absorbent article (1) according to any of claims 1-10, wherein an extra non-elastic zone (7') separates the first elastic zones (6) from the back section (10).

12. The absorbent article (1) according to any of claims 1-11, wherein the first and the second elastic zones (6, 8) have a shape selected from the list comprising triangular, square and circular shape, preferably said elastic zones (6, 8) have a rectangular shape.

13. The absorbent article (1) according to any of claims 1-12, wherein the first elastic zone has an elastic resistance comprised between 10 and 80 N.

14. The absorbent article (1) according to any of claims 1-13, wherein the second elastic zone has an elastic resistance comprised between 0 and 60 N.
